# EUROPEAN PATENT SPECIFICATION

(11) **EP 4 143 577 B1**
(45) Date of publication and mention of the grant of the patent: **10.12.2025**
(21) Application number: 21733006.7
(22) Date of filing: 10.05.2021
(51) Int. Cl.: G01N 33/569

(54) **METHODS AND KITS FOR DETECTING ADENO-ASSOCIATED VIRUSES**
VERFAHREN UND KITS ZUM NACHWEIS VON ADENO-ASSOZIIERTEN VIREN
MÉTHODES ET KITS DE DÉTECTION DE VIRUS ADÉNO-ASSOCIÉS

(30) Priority: 12.05.2020 US 202063023629 P
(43) Date of publication of application: 08.03.2023
(73) Proprietor: Lonza Houston Inc., Pearland, TX 77047 (US)
(72) Inventor: WANG, Peng, Pearland, Texas 77047 (US); GU, Bingnan, Pearland, Texas 77047 (US); SETH, Anandita, Pearland, Texas 77047 (US)
(74) Representative: Greiner, Elisabeth
(86) International application number: PCT/US2021/031577
(87) International publication number: WO 2021/231296

(56) References cited:
- WO-A1-2021/067874
- WO-A2-2019/067982
- ERIC ZINN ET AL: "In Silico Reconstruction of the Viral Evolutionary Lineage Yields a Potent Gene Therapy Vector", CELL REPORTS, vol. 12, no. 6, 1 August 2015 (2015-08-01), US, pages 1056 - 1068, XP055524241, ISSN: 2211-1247, DOI: 10.1016/j.celrep.2015.07.019
- LUKAS D LANDEGGER ET AL: "A synthetic AAV vector enables safe and efficient gene transfer to the mammalian inner ear", NATURE BIOTECHNOLOGY, vol. 35, no. 3, 6 February 2017 (2017-02-06), New York, pages 280 - 284, XP0055675069, ISSN: 1087-0156, DOI: 10.1038/nbt.3781
- B. L. GURDA ET AL: "Mapping a Neutralizing Epitope onto the Capsid of Adeno-Associated Virus Serotype 8", JOURNAL OF VIROLOGY, vol. 86, no. 15, 16 May 2012 (2012-05-16), US, pages 7739 - 7751, XP055590574, ISSN: 0022-538X, DOI: 10.1128/JVI.00218-12
- GRIMM D ET AL: "TITRATION OF AAV-2 PARTICLES VIA A NOVEL CAPSID ELISA: PACKAGING OF GENOMES CAN LIMIT PRODUCTION OF RECOMBINANT AAV-2", GENE THERAPY, NATURE PUBLISHING GROUP, LONDON, GB, vol. 6, no. 7, 1 July 1999 (1999-07-01), pages 1322 - 1330, XP000892113, ISSN: 0969-7128, DOI: 10.1038/SJ.GT.3300946
- CUI MENGTIAN ET AL: "A Generic Method for Fast and Sensitive Detection of Adeno-Associated Viruses Using Modified AAV Receptor Recombinant Proteins", MOLECULES, vol. 24, no. 21, 3 November 2019 (2019-11-03), pages 3973, XP055821357, Retrieved from the Internet <URL:https://www.ncbi.nlm.nih.gov/pmc/articles/PMC6864843/pdf/molecules-24-03973.pdf> DOI: 10.3390/molecules24213973

## Description

### FIELD OF THE INVENTION

The present disclosure provides methods and kits for detecting adeno-associated viruses of serotype Anc80. Detection methods include HPLC- and ELISA-based methods.

### BACKGROUND

Adeno-associated viruses (AAVs) have emerged as an important tool for gene therapy. In general, an AAV for gene therapy is an assembled viral particle, e.g., that lacks the native viral genes and only contains a sequence of interest, such as a gene of interest (GOI), resulting in a recombinant AAV (rAAV). The gene of interest is then delivered by the rAAV particle into the host cell. A typical process for producing a rAAV particle involves delivering at least two plasmids into a producer cell: a first plasmid containing the gene of interest flanked by inverted terminal repeat (ITR) sequences, which are found in the native AAV genome (the ITR/GOI plasmid); a second plasmid containing the AAV rep/cap genes to be expressed *in trans* for virus assembly (the rep/cap plasmid); and often a third plasmid containing helper genes from either adeno or herpes viruses (the helper plasmid). The producer cell then produces an assembled rAAV particle containing the ITR/GOI plasmid (i.e., a "packaged" rAAV), and the packaged rAAV can then be isolated and purified from the producer cell.

Purification of rAAVs typically involves: harvesting the producer cells; lysing the producer cells; subjecting the cell lysate to gradient purification (e.g., using a iodixanol gradient) and/or fast protein liquid chromatography (FPLC), which can be ion exchange, size exclusion, and/or affinity based; and buffer exchange and concentration, e.g., using tangential flow filtration. After purification, the rAAV titer is typically measured. Recombinant AAVs and methods of production and purification are further described, e.g., in Naso et al., BioDrugs 31(4):317-334 (2017) and WO 2018/150269. WO 2019/067982 discloses methods of purifying AAV particles including the use of affinity chromatography with a chromatography support on which an anti-AAV8 antibody or an AAV8-binding fragment is immobilized.

Purification of AAVs, e.g., rAAVs from producer cells, can be a time-consuming and labor-intensive process. Since virus titration is typically not performed until after a substantially purified sample is obtained, e.g., after most or all of the purification steps, a low yield batch (e.g., low number of viral particles and/or low ratio of full/empty capsids) is not detected until a substantial amount of time and reagents have been spent, greatly increasing costs and reducing production efficiency.

A further challenge associated with AAV production is to develop optimized processes for different viral vectors on a large scale. The iterative optimization of both upstream and downstream processes utilize analytic tools to characterize and quantify viral products for in-process samples.

### SUMMARY OF THE INVENTION

In some embodiments, the disclosure provides a method for detecting adeno-associated virus serotype Anc80 in a sample, comprising: (a) contacting the sample with: (i) a capture reagent that specifically binds an adeno-associated virus (AAVx) or an adeno-associated virus serotype 8 (AAV8); and (ii) a detection reagent that binds a broad array of adeno-associated virus serotypes (AAVx), wherein each of the capture reagent and the detection reagent is capable of binding the Anc80, and wherein the capture and detection reagent are antibodies or antigen-binding fragments thereof; (b) forming a binding complex comprising the capture reagent, the Anc80, and the detection reagent; and (c) detecting the binding complex, thereby detecting the Anc80 in the sample, wherein the capture reagent is immobilized on a surface.

In some embodiments, the capture reagent comprises biotin, and the surface comprises avidin or streptavidin. In some embodiments, the surface is a multi-well plate, and the capture reagent is immobilized in a well of the multi-well plate. In some embodiments, the capture reagent and/or the detection reagent bind(s) to a capsid protein of the Anc80. More specifically, the capture reagent may bind to VP3 of Anc80, preferably wherein the capture reagent binds to an epitope within residues 580 to 600 of the Anc80 VP3, more preferably wherein the capture reagent binds to LQSANT (SEQ ID NO: 1). In some embodiments, the detection reagent comprises a binding partner of a detectable moiety, optionally wherein the detection reagent comprises biotin, and the detectable moiety comprises horseradish peroxidase conjugated to avidin or streptavidin.

In additional embodiments, the disclosure provides a kit for detecting adeno-associated virus serotype Anc80 in a sample, comprising: (a) a capture reagent that specifically binds an adeno-associated virus serotype 8 (AAV8); and (b) a detection reagent that binds a broad array of adeno-associated virus serotypes (AAVx); and (c) a capture surface, wherein the capture reagent is immobilized on the capture surface, or wherein the capture reagent is configured to be immobilized to the capture surface, wherein each of the capture reagent and the detection reagent is capable of binding the Anc80, and wherein the capture and detection reagent are antibodies or antigen-binding fragments thereof.

In some embodiments, the capture reagent, the detection reagent, or both, are (i) lyophilized or (ii) provided in solution. In some embodiments, the capture reagent comprises biotin, and the capture surface comprises avidin or streptavidin attached thereto. In some embodiments, the capture surface is a multi-well plate, and the capture reagent is immobilized on a well of the multi-well plate. In some embodiments, the capture reagent and/or the detection reagent bind(s) to a capsid protein of the Anc80. More specifically, the capture reagent may bind to VP3 of Anc80, preferably wherein the capture reagent binds to an epitope within residues 580 to 600 of the Anc80 VP3, more preferably wherein the capture reagent binds to LQSANT (SEQ ID NO: 1). In some embodiments, the kit further comprises a detectable moiety, and the detection reagent comprises a binding partner of the detectable moiety, optionally wherein the detection reagent comprises biotin, and the detectable moiety comprises horseradish peroxidase conjugated to avidin or streptavidin. In some embodiments, the kit further comprises one or more of a buffer, an assay stop solution, a calibration reagent, a detectable moiety capable of binding to the detection reagent, and a detectable substrate.

### BRIEF DESCRIPTION OF THE DRAWINGS

The following drawings form part of the present specification and are included to further demonstrate exemplary embodiments of certain aspects of the present invention.
FIG. 1 illustrates an exemplary AAV purification process. The AAV producer cells are grown in a bioreactor and collected by depth filtration. The AAVs are isolated and purified by chromatography.
FIGS. 2A-2C show results of HPLC chromatography experiments described in Example 1. FIGS. 2A, 2B, and 2C show respectively the standard curves generated from samples of Anc80.CMV.eGFP, AAV2.CMV.eGFP, and AAV8.CMV.eGFP using the same HPLC column.
FIG. 3 shows the results of HPLC chromatography experiments described in Example 1. FIG. 3 shows HPLC chromatograms of samples obtained during or after various steps of the AAV purification process, i.e., cell suspension, cell lysate, clarification of lysate, tangential flow filtration (TFF) retentate, and final filtration.
FIG. 4 shows the results of AAV titer calculations described in Example 1. Full/empty capsid ratios were determined using capsid titer measured by HPLC (calculated from the results in FIG. 3) and genome titer measured by ddPCR.
FIG. 5 shows a standard curve of an ELISA performed according to Example 2, with samples of Anc80.CMV.eGFP.
FIG. 6 shows a 4-parameter logic regression calculation to determine the curve fit of the standard curve shown in FIG. 5.

### DETAILED DESCRIPTION OF THE INVENTION

The present disclosure relates to methods and kits for detecting an adeno-associated virus of serotype Anc80.

Unless otherwise defined herein, scientific and technical terms used in the present disclosure shall have the meanings that are commonly understood by one of ordinary skill in the art.

Unless otherwise required by context, singular terms shall include pluralities and plural terms shall include the singular. As used herein, "a" or "an" may mean one or more. As used herein, when used in conjunction with the word "comprising," the words "a" or "an" may mean one or more than one. As used herein, "another" or "a further" may mean at least a second or more.

Throughout this application, the term "about" is used to indicate that a value includes the inherent variation of error for the method/device being employed to determine the value, or the variation that exists among the study subjects. Typically, the term "about" is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20%, depending on the situation. In some embodiments, one of skill in the art will understand the level of variability indicated by the term "about," due to the context in which it is used herein. It should also be understood that use of the term "about" also includes the specifically recited value.

As used herein, the terms "comprising" (and any variant or form thereof, such as "comprise" and "comprises"), "having" (and any variant or form thereof, such as "have" and "has"), "including" (and any variant or form thereof, such as "includes" and "include") or "containing" (and any variant or form thereof, such as "contains" and "contain") are inclusive or open-ended and do not exclude additional, unrecited elements or method steps. It is contemplated that any embodiment discussed in this specification can be implemented with respect to any method and/or kit of the present disclosure.

The use of the term "for example" and its corresponding abbreviation "e.g." means that the specific terms recited are representative examples and embodiments of the disclosure that are not intended to be limited to the specific examples referenced or cited unless explicitly stated otherwise.

As used herein, "between" is a range inclusive of the ends of the range. For example, a number between x and y explicitly includes the numbers x and y, and any numbers that fall within x and y.

As referred to herein, "adeno-associated virus" or "AAV" refers to a small sized, replicative-defective nonenveloped virus or viral particle containing a single stranded DNA of the family *Parvoviridae* and the genus *Dependoparvovirus.* Adeno-associated virus also include ancestral AAVs (Anc AAVs). Non-limiting examples of AAV serotypes include Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. In addition to these serotypes, AAV pseudotypes have been developed. An AAV pseudotype contains the capsid of a first serotype and the genome of a second serotype (e.g., the pseudotype AAV2/5 corresponds to an AAV with the genome of serotype AAV2 and the capsid of AAV5). Methods of producing derivatives, modifications, and/or pseudotypes of AAVs are described in, e.g., Asokan et al., Mol. Ther. 20(4):699-708 (2012).

As used herein, the "rep" gene refers to the art-recognized region of the AAV genome that encodes the replication proteins of the AAV, which are collectively required for replicating the viral genome. Rep also refers to functional homologues of AAV genes, such as the human herpesvirus 6 (HHV-6) rep gene, which is also known to mediate AAV DNA replication. AAVs described herein may encode a rep coding region.

As used herein, the "cap" gene refers to the art-recognized region of the AAV genome that encodes the capsid proteins of the AAV. Illustrative and non-limiting examples of capsid proteins are the AAV capsid proteins VP1, VP2, and VP3. The AAV capsid proteins interact to form an AAV capsid. Cap genes used in the present disclosure can refer to the cap genes from any AAV serotype or a combination of serotypes.

As used in the context of AAVs, the term "recombinant" means that the AAV is the product of one or more procedures that results in an AAV that is distinct from a naturally-occurring AAV. A recombinant AAV, or rAAV, refers to a virus or viral particle comprising at least one AAV capsid protein and an encapsidated polynucleotide rAAV vector comprising a heterologous polynucleotide (i.e., a polynucleotide other than a naturally-occurring AAV genome, e.g., comprising a transgene, or gene of interest,to be delivered into a cell such as a mammalian cell). The rAAV particle can be any serotype, pseudotype, or derivative described herein (e.g., Anc80, AAV1, AAV-2, AAV3, AAV4, AAV5, AAV6, AAV7, AAV8, AAV9, AAV10, AAV11, AAV12, AAV13, AAV14, AAV15, AAV16, or derivatives, modifications, and/or pseudotypes thereof). The rAAV particle can also include a combination of serotypes.

As used herein, the term "titer" or "viral titer" refers to the concentration of a virus, e.g., an AAV provided herein. Viral titer can be expressed as the number of viral particles or the number of infectious units per mL. Viral titer may be determined by measuring the concentration of a viral component, e.g., a viral capsid or genome.

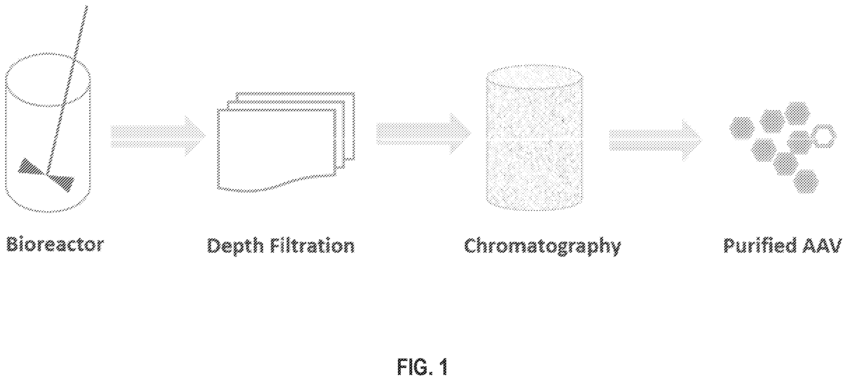

As described herein, purification of AAVs, e.g., rAAVs from producer cells, can be a time-consuming and labor-intensive process. An exemplary process for rAAV production and purification is illustrated in FIG. 1. In FIG. 1, producer cells are grown in a bioreactor to a desired cell density. The cells are collected by depth filtration and subjected to chromatography to obtain purified AAVs. Since virus titration is typically not performed until after a substantially purified sample is obtained, e.g., after most or all of the purification steps, a low yield batch (e.g., low number of viral particles and/or low ratio of full/empty capsids) is not detected until a substantial amount of time and reagents have been spent, greatly increasing costs and reducing production efficiency.

Described but not claimed herein is an analytical method to determine the AAV titer produced by a producer cell. The method may comprise measuring the AAV capsid titer. The method may comprise measuring the AAV genome titer. The same method may be used to detect different AAV serotypes, thereby providing an efficient and simplified procedure over current methods that require serotype-specific reagents, which may have different protocols. For example, for manufacturers of different serotypes of AAVs, the ability to utilize a single, streamlined process for detecting of any AAV serotype can reduce the number of equipment and maintenance thereof, allow equipment to be utilized more efficiently, lower reagent, processing, and labor costs, simplify personnel training, reduce the amount of different certifications, improve manufacturability and scaling, etc.

The method may be capable of detecting and/or titrating an AAV (e.g., Anc80) during the AAV purification process from a producer cell, as described herein. The method is advantageously capable of sensitive and specific detection and/or titration of AAV (e.g., Anc80), even in crude, unpurified samples, that may contain interfering components such as producer cell proteins and cellular debris, thereby providing a simple and efficient in-process AAV test. For example, the method can be performed in 90 min or less, 75 min or less, 60 min or less, or 45 in or less. The method may be capable of detecting and/or titrating an AAV (e.g., Anc80) in cell lysate. The method may be capable of detecting and/or titrating an AAV (e.g., Anc80) in a partially purified cell lysate. "Partially purified" cell lysate refers to a cell lysate that has been subjected to one or more downstream purification process steps after cell lysis, e.g., centrifugation, chromatography, buffer exchange, or filtration. The method may be capable of detecting and/or titrating an AAV (e.g., Anc80) in a purified sample, e.g., a sample that has been purified from an AAV producer cell as described herein. The purified sample may be free or substantially free of non-AAV particles.

### Chromatography Methods

Disclosed but not claimed herein is an analytical method to determine the AAV titer produced by a producer cell. The method may be used to determine AAV titer at different points during the production process, e.g., at different time points during producer cell growth and/or during the AAV isolation and purification process described herein. The method may be capable of detecting and/or titrating AAV without lysing the producer cells. The method may comprise measuring the AAV capsid titer.

Disclosed but not claimed herein is a method of determining capsid titer of an adeno-associated virus in a cell suspension or cell lysate the method comprising: (a) subjecting the cell suspension or cell lysate to high-performance liquid chromatography (HPLC), wherein the HPLC is performed with a resin that binds to a viral particle (VP) of the adeno-associated virus; and (b) detecting the VP in the cell suspension or cell lysate, thereby determining capsid titer of the adeno-associated virus.

The resins described herein may be able to bind to capsid protein(s) of the viral particle.

The detection of the VP may occur spectrophotometrically, including for example by the use of absorbance measurements. Devices for measuring the absorbance of a solution following HPLC are well known and the art and readily configured to use as either part of an HPLC apparatus, or separately from an HPLC column. The detection of the VP of the adeno-associated virus may occur by obtaining the absorbance of the sample at about 270-290 nm, more suitably at about 280 nm, or specifically at 280 nm. This measurement can then be compared to calibration curve (either manually or as part of the instrument measurement) to provide an output of the amount of viral capsid in the sample.

The cell suspension may comprise producer cells suspended in growth media and/or buffer. The cell lysate may be an unpurified cell lysate, i.e., a cell lysate that has not been subjected to any downstream purification process after cell lysis. The cell lysate may be a partially purified cell lysate, e.g., a cell lysate that has been subjected to one or more downstream purification processes after cell lysis, as described herein. The cell lysate may comprise one or more impurities, which can include proteins, nucleic acids, polysaccharides, lipids, or other cellular components of the producer cell. The impurity may comprise a viral component that is not part of the assembled AAV particle. The impurity may comprise a partially assembled AAV particles. The impurity may comprise a misfolded viral protein or a malformed viral particle.

As described herein, the AAV may comprise a fluorescent moiety. The fluorescent moiety may be a fluorescent dye. The fluorescent moiety may be incorporated into the AAV particle, e.g., incorporated into the capsid. The fluorescent moiety may be incorporated into a nucleic acid in the AAV. The fluorescent moiety may be incorporated into a protein or polypeptide in the AAV. The fluorescent moiety may be a fluorescent protein. The AAV may express the fluorescent protein. The fluorescent protein may be green fluorescent protein (GFP), blue fluorescent protein (BFP), red fluorescent protein (RFP), yellow fluorescent protein (YFP), cyan fluorescent protein (CFP), mCherry, mApple, mTurquoise, mVenus, mKO2, mKate2, or any variant or derivative thereof (e.g., eGFP). Additional fluorescent moieties and tags are known in the art, and include for example red fluorescent proteins such as TagRFP, mKate2, mRuby2, and Fusion Red, as well as other fluorescence proteins including for example monomerized (V206K) superfolder GFP, mTurquoise, Cerulean3, enhanced blue FP 2 (EBFP2), TagBFP, mNeonGreen, and monomerized Venus (A206K), etc. Where a fluorescence moiety or tag is utilized with the adeno-associated virus, the detection suitably comprises fluorescence detection. Devices for such fluorescence detection are known in the art and can readily be combined with HPLC devices and apparatuses. Fluorescent moieties are further described, e.g., in Jensen, The Anatomical Record 295(12):2031-2036 (2012). The AAV may be detected using fluorescence, e.g., via a fluorescence detector attached to the HPLC. Fluorescence detection, e.g., of VP, has higher sensitivity as compared with detection of absorbance at 260 nm and/or 280 nm. Fluorescence detection can also provide a simple and straightforward output compared with A260/A280 measurements, which require a ratio calculation. The limit of detection of the methods described herein that utilize fluorescence detection or absorbance measurements may be about 10⁶, about 10⁷, about 10⁸, about 10⁹, or about 10¹⁰ viral particles.

The HPLC may be performed with the resin contained in a column having a volume of about 0.1 mL to about 5.0 mL, about 0.1 mL to about 4.0 mL, about 0.1 mL to about 3.0 mL, about 0.1 mL to about 2.0 mL, about 0.1 mL to about 1.0 mL, about 0.1 mL to about 0.9 mL, about 0.2 mL to about 0.8 mL, about 0.3 mL to about 0.7 mL, or about 0.4 mL to about 0.6 mL. The HPLC may be performed with the resin contained in a column having a volume of about 0.1 mL, about 0.2 mL, about 0.3 mL, about 0.4 mL, about 0.5 mL, about 0.6 mL, about 0.7 mL, about 0.8 mL, about 0.9 mL, about 1.0 mL, about 1.5 mL, about 2.0 mL, about 2.5 mL, about 3.0 mL, about 3.5 mL, about 4.0 mL, about 4.5 mL, or about 5.0 mL.

The resin may be an affinity resin. Suitably, the resin comprises an antibody or variant thereof, including an antigen/epitope-binding portion thereof, an antibody fragment or derivative, an antibody analogue, an engineered antibody, or a substance that binds to an antigen in a similar manner to an antibody. The resin may comprise at least one heavy or light chain complementarity determining region (CDR) of an antibody. The resin may comprise at least two CDRs from one or more antibodies. The resin may comprise an antibody or antigen-binding fragment thereof.

The resin may bind an adeno-associated virus of any serotype or a broad array of AAV serotypes. As described herein, AAVx refers to such broad array of AAV serotypes. The resin may be capable of specifically binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. The AAV may comprise Anc80. The resin may bind a capsid protein of the viral particle (VP) of the AAV. The resin may bind an assembled AAV particle, and suitably, the resin binds a conformational epitope on the AAV capsid.

The AAV titer (e.g., Anc80 titer) may be determined by measuring the peak area on the HPLC chromatogram. The method may further comprise measuring the AAV genome titer (e.g., Anc80 titer) by digital droplet PCR (ddPCR). Titration of viruses, e.g., AAVs, by ddPCR is described in Furuta-Hanawa et al., Hum. Gene Ther. Methods 30(4):127-136 (2019). The method may further comprise determining a ratio of full to empty capsids in the cell suspension or cell lysate based on the result of the AAV titers measured by HPLC and ddPCR.

As described herein, it has been surprisingly found that HPLC methods can be utilized to determine capsid titer or AAV serotypes, including Anc80, without the need for first purifying a cellular sample, and with the use of a small sample size (e.g., 10s-100s of mL of sample). This allows for a rapid and simple method of viral titer determination directly during processing, allowing for a fast determination regarding downstream processing.

The HPLC methods described herein can be utilized in combination with any bioreactor as well as any volume of bioreactor process. Exemplary reactor(s) include but are not limited to stirred tank, airlift, fiber, microfiber, hollow fiber, ceramic matrix, fluidized bed, fixed bed, and/or spouted bed bioreactors. As used herein, "reactor" can include a fermenter or fermentation unit, or any other reaction vessel and the term "reactor" is used interchangeably with "fermenter." The term fermenter or fermentation refers to both microbial and mammalian cultures. For example, an example bioreactor unit can perform one or more, or all, of the following: feeding of nutrients and/or carbon sources, injection of suitable gas (e.g., oxygen), inlet and outlet flow of fermentation or cell culture medium, separation of gas and liquid phases, maintenance of temperature, maintenance of oxygen and CO2 levels, maintenance of pH level, agitation (e.g., stirring), and/or cleaning/sterilizing. Example reactor units, such as a fermentation unit, may contain multiple reactors within the unit, for example the unit can have 1, 2, 3, 4, 5, 10, 15, 20, 25, 30, 35, 40, 45, 50, 60, 70, 80, 90, or 100, or more bioreactors in each unit and/or a facility may contain multiple units having a single or multiple reactors within the facility. In various embodiments, the bioreactor can be suitable for batch, semi fed-batch, fed-batch, perfusion, and/or a continuous fermentation processes. Any suitable reactor diameter can be used. The bioreactor can have a volume between about 100 mL and about 50,000 L. Non-limiting examples include a volume of 100 mL, 250 mL, 500 mL, 750 mL, 1 liter, 2 liters, 3 liters, 4 liters, 5 liters, 6 liters, 7 liters, 8 liters, 9 liters, 10 liters, 15 liters, 20 liters, 25 liters, 30 liters, 40 liters, 50 liters, 60 liters, 70 liters, 80 liters, 90 liters, 100 liters, 150 liters, 200 liters, 250 liters, 300 liters, 350 liters, 400 liters, 450 liters, 500 liters, 550 liters, 600 liters, 650 liters, 700 liters, 750 liters, 800 liters, 850 liters, 900 liters, 950 liters, 1000 liters, 1500 liters, 2000 liters, 2500 liters, 3000 liters, 3500 liters, 4000 liters, 4500 liters, 5000 liters, 6000 liters, 7000 liters, 8000 liters, 9000 liters, 10,000 liters, 15,000 liters, 20,000 liters, and/or 50,000 liters. Additionally, suitable reactors can be multi-use, single-use, disposable, or non-disposable and can be formed of any suitable material including metal alloys such as stainless steel (e.g., 316L or any other suitable stainless steel) and Inconel, plastics, and/or glass.

### Immunoassay Method

The disclosure provides an immunoassay method for detecting and/or titrating an adeno-associated virus (AAV) in a sample. More specifically, the disclosure provides a method for detecting adeno-associated virus serotype Anc80 in a sample, comprising: (a) contacting the sample with: (i) a capture reagent that specifically binds an adeno-associated virus (AAVx) or an adeno-associated virus serotype 8 (AAV8); and (ii) a detection reagent that binds a broad array of adeno-associated virus serotypes (AAVx), wherein each of the capture reagent and the detection reagent is capable of binding the Anc80, and wherein the capture and detection reagent are antibodies or antigen-binding fragments thereof; (b) forming a binding complex comprising the capture reagent, the Anc80, and the detection reagent; and (c) detecting the binding complex, thereby detecting the Anc80 in the sample, wherein the capture reagent is immobilized on a surface. The method may be used to determine AAV titer at different points during the production process, e.g., at different time points during producer cell growth and/or during the AAV isolation and purification process described herein. In some embodiments, the method is capable of detecting and/or titrating AAV without lysing the producer cells. In some embodiments, the method comprises measuring the AAV capsid titer.

The method is an immunoassay (e.g., an enzyme-linked immunosorbent assay or ELISA) for detecting and/or titrating an AAV (including Anc80) in a sample. Immunoassays provide numerous advantages, such as high specificity and sensitivity, and can also be conducted in a high throughput format to assess multiple samples in one experiment. In some embodiments, the immunoassay is capable of detecting and/or titrating multiple AAV serotypes using the same capture and/or detection reagents. For example, the immunoassay can be conducted in a multi-well plate, and each well (or each group of wells) can correspond to a different AAV serotype.

In some embodiments, the sample is a cell suspension, i.e., comprising producer cells suspended in growth media and/or buffer. In some embodiments, the sample is an unpurified cell lysate, i.e., a cell lysate that has not been subjected to any purification process after cell lysis. In some embodiments, the sample is a partially purified cell lysate, e.g., a cell lysate that has been subjected to one or more purification processes after cell lysis, as described herein. In some embodiments, the sample comprises one or more impurities. In some embodiments, the impurity comprises a protein, nucleic acid, polysaccharide, lipid, or other cellular component of the producer cell. In embodiments, the impurity comprises a viral component that is not part of the assembled AAV particle. In other embodiments, the impurity comprises a partially assembled AAV particle. In some embodiments, the impurity comprises a misfolded viral protein or a malformed viral particle.

Anc80 is a predicted ancestor of AAV serotypes 1, 2, 8, and 9 and was reconstructed by Zinn et al. as a potent gene therapy vector (see Zinn et al., Cell Reports 12:1056-1068 (2015) and Landegger et al., Nature Biotechnol. 35:280-284 (2017)). Anc80 clones include, e.g., Anc80L27, Anc80L65, Anc80L121. In some embodiments, the method provided herein is capable of detecting Anc80L27, Anc80L65, and Anc80L121.

### Capture and Detection Reagents

According to the invention, the capture reagent is an antibody or antigen-binding fragment thereof. In some embodiments, the capture reagent is a monoclonal antibody.

The capture reagent is immobilized on a surface. Suitable surfaces include, e.g., a particle (such as a bead), including a column prepared from such particles, as well as a plastic substrate such as a multi-well plate. In some embodiments, the surface comprises a multi-well plate, and the capture reagent is immobilized in a well of the multi-well plate. In some embodiments, the surface comprises a particle, and the capture reagent is immobilized on the particle. In some embodiments, the capture reagent comprises a conjugation moiety that is capable of reacting with its corresponding conjugation partner on the surface. Exemplary conjugation moiety-conjugation partner pairs include, but are not limited to, a receptor-ligand pair, complementary oligonucleotides, or cross-reactive moieties such as, e.g., thiol and maleimide or iodoacetamide; aldehyde and hydrazide; or azide and alkyne or cycloalkyne. In some embodiments, the capture reagent comprises biotin, and the surface comprises avidin or streptavidin.

According to the invention, the capture reagent binds Anc80. In embodiments, the capture reagent binds a capsid protein (VP) of the Anc80. In some embodiments, the capture reagent binds an assembled AAV particle. In some embodiments, the capture reagent binds a conformational epitope on the AAV capsid.

According to the invention, the capture reagent also specifically binds AAV8. Thus, the capture reagent is capable of binding AAV8 and Anc80.

In some embodiments, the capture reagent binds to a capsid protein of a viral particle (VP) of AAV8 or a protein of Anc80. Suitably, the capture reagent binds the VP3 protein of AAV8 or the VP3 protein Anc80. In embodiments, the capture reagent binds to an epitope within residues 575-610, or within residues 580-600, or within residues 580-595 of the VP3 of AAV8 or the VP3 protein of Anc80. In suitable embodiments, the capture reagent binds to an epitope within residues 586-591 of the AAV8 VP3 protein. In other embodiments, the capture reagent binds to an epitope within residues 589-594 of the Anc80 VP3 protein. In some embodiments, the capture reagent binds to the sequence LQSANT (SEQ ID NO: 1). In other embodiments, the capture reagent binds to the sequence LQQQNT (SEQ ID NO:2). In some embodiments, the capture reagent is AAV8 antibody clone ADK8.

According to the invention, the detection reagent is an antibody or antigen-binding fragment thereof. In some embodiments, the detection reagent is a monoclonal antibody.

The detection reagent binds an adeno-associated virus of any serotype (AAVx). In some embodiments, the detection reagent is capable of binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. According to the invention, the detection reagent binds Anc80. In some embodiments, the detection reagent binds a capsid protein of a VP of the Anc80. In some embodiments, the detection reagent binds an assembled AAV particle. In some embodiments, the detection reagent binds a conformational epitope on the AAV capsid.

### Binding Complex Formation

In some embodiments, the binding complex comprising the capture reagent, the AAV (e.g., Anc80), and the detection reagent is formed in a single step. In other embodiments, the binding complex comprising the capture reagent, the AAV (e.g., Anc80), and the detection reagent is formed in one or more steps. In some embodiments, the binding complex is formed in solution, then immobilized to the surface. In some embodiments, the binding complex is formed by binding the AAV (e.g., Anc80) to the capture reagent immobilized on the surface, then binding the detection reagent to the AAV (e.g., Anc80) to form the binding complex on the surface. In some embodiments, the binding complex is formed by binding to the AAV (e.g., Anc80) to the capture reagent immobilized on the surface and to the detection reagent simultaneously. In other embodiments, the binding complex is formed by binding the AAV (e.g., Anc80) to the detection reagent in solution, then binding the AAV-detection reagent complex to the capture reagent on the surface. In additional embodiments, the binding complex is formed by binding the AAV (e.g., Anc80) to the capture reagent and the detection reagent in solution, then immobilizing the capture reagent to the surface as described herein.

### Detection

In embodiments, the binding complex is detected via a detectable moiety. For example, the detectable moiety is measured by spectrophotometry (color change), light scattering, optical absorbance, fluorescence, chemiluminescence, electrochemiluminescence, bioluminescence, phosphorescence, radioactivity, magnetic field, or combination thereof. In embodiments, the AAV titer (e.g., Anc80 titer) is determined by measuring the detectable moiety. In some embodiments, the detectable moiety is detectable in the presence of a substrate. Suitably, the detectable moiety is an enzyme that cleaves a substrate, and the detecting comprises detecting the cleaved substrate. In some embodiments, the detectable moiety comprises horseradish peroxidase (HRP), alkaline phosphatase (AP), glucose oxidase (GO), or beta galactosidase (BGAL or β-gal). Non-limiting examples of HRP substrates include, e.g., 3,3',5,5'-tetramethylbenzidine (TMB), 3,3'-diaminobenzidine (DAB), 2,2'-azino-bis(3-ethylbenzothiazoline-6-sulfonic acid) (ABTS), o-phenylenediamine dihydrochloride (OPD), AMPLEX^{®} Red; 3-amino-9-ethylcarbazole (AEC), homovanillic acid, luminol, and the SUPERSIGNAL^{™} ELISA, QUANTABLU^{™}, and QUANTARED^{™} substrates from ThermoFisher. Non-limiting examples of AP substrates include, e.g., p-nitrophenyl phosphate (PNPP) and the CDP-STAR^{™} and DYNALIGHT^{™} substrates from ThermoFisher. GO substrates include, e.g., glucose. BGAL substrates include, e.g., o-nitrophenyl-β-D-galactopyranoside (ONPG).

In some embodiments, the detection reagent comprises a detectable moiety, and detecting the binding complex comprises measuring the detectable moiety of the detection reagent. In embodiments, the detection reagent comprises a binding partner of a detectable moiety, and detecting the binding complex comprises contacting the detection reagent with the detectable moiety and measuring the detectable moiety bound to the detection reagent. In additional embodiments, the detection reagent and detectable moiety each comprises a binding partner in a binding pair. Suitable binding pairs are known in the art can include, but are not limited to, a receptor-ligand pair, complementary oligonucleotides, or cross-reactive moieties such as, e.g., thiol and maleimide or iodoacetamide; aldehyde and hydrazide; or azide and alkyne or cycloalkyne. In some embodiments, the detection reagent comprises biotin, and the detectable moiety comprises avidin or streptavidin. In some embodiments, the detection reagent comprises biotin, and the detectable moiety comprises horseradish peroxidase (HRP) conjugated to avidin or streptavidin. Suitably, detecting the binding complex comprises: binding the binding complex to HRP (e.g., via the biotin-avidin/streptavidin interaction on the detection reagent and HRP), contacting the HRP with a chromogenic or chemiluminescent HRP substrate, and detecting a change in color or a chemiluminescence signal. In some embodiments, the AAV titer (e.g., Anc80 titer) is determined by measuring the change in color and/or chemiluminescence signal.

### Kits

In some embodiments, the disclosure provides a kit for conducting the methods described herein, i.e., an immunoassay kit. More specifically, the disclosure provides a kit for detecting adeno-associated virus serotype Anc80 in a sample, comprising: (a) a capture reagent that specifically binds an adeno-associated virus serotype 8 (AAV8); and (b) a detection reagent that binds a broad array of adeno-associated virus serotypes (AAVx); and (c) a capture surface, wherein the capture reagent is immobilized on the capture surface, or wherein the capture reagent is configured to be immobilized to the capture surface, wherein each of the capture reagent and the detection reagent is capable of binding the Anc80, and wherein the capture and detection reagent are antibodies or antigen-binding fragments thereof. The kits described herein also suitably include instructions for carrying out the various methods, including the ELISA-based methods, described herein.

Capture reagents and detection reagents are further described herein. According to the invention, the capture and detection reagents are antibodies or antigen-binding fragments thereof.

The kit comprises a capture surface. Capture surfaces can be any of the surfaces described herein. In some embodiments, the capture surface comprises a particle. In some embodiments, the capture surface comprises a plastic substrate such as a multi-well plate. In some embodiments, the capture reagent is immobilized on the capture surface. In some embodiments, the capture surface is a multi-well plate, and the capture reagent is immobilized on a well of the multi-well plate.

In some embodiments, the capture reagent and the capture surface are provided separately, and the kit further comprises a reagent for immobilizing the capture reagent to the capture surface. In some embodiments, the capture reagent comprises a conjugation moiety, and the capture surface comprises a conjugation partner thereof. Exemplary conjugation moiety-conjugation partner pairs include, but are not limited to, a receptor-ligand pair, complementary oligonucleotides, or cross-reactive moieties such as, e.g., thiol and maleimide or iodoacetamide; aldehyde and hydrazide; or azide and alkyne or cycloalkyne. In some embodiments, the capture reagent comprises biotin, and the capture surface comprises avidin or streptavidin attached thereto.

According to the invention, the capture reagent binds Anc80. In some embodiments, the capture reagent binds a capsid protein (VP) of the Anc80. In some embodiments, the capture reagent binds an assembled AAV particle. In some embodiments, the capture reagent binds a conformational epitope on the AAV capsid.

According to the invention, the capture reagent also specifically binds AAV8.Thus, the capture reagent is capable of binding AAV8 and Anc80. In some embodiments, the capture reagent binds to a capsid protein of a viral particle (VP) of AAV8 or a capsid protein of Anc80. In some embodiments, the capture reagent binds the VP3 protein of AAV8 or the VP3 protein Anc80. In some embodiments, the capture reagent binds to an epitope within residues 575-610, or within residues 580-600, or within residues 580-595 of the VP3 of AAV8 or the VP3 protein of Anc80. In some embodiments, the capture reagent binds to an epitope within residues 586-591 of the AAV8 VP3 protein. In some embodiments, the capture reagent binds to an epitope within residues 589-594 of the Anc80 VP3 protein. In some embodiments, the capture reagent binds to the sequence LQSANT (SEQ ID NO: 1). In some embodiments, the capture reagent binds to the sequence LQQQNT (SEQ ID NO:2). In some embodiments, the capture reagent is AAV8 antibody clone ADK8.

The detection reagent binds an adeno-associated virus of any serotype (AAVx). In some embodiments, the detection reagent is capable of binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127.According to the invention, the detection reagent binds Anc80. In some embodiments, the detection reagent binds a capsid protein (VP) of the Anc80. In some embodiments, the detection reagent binds an assembled AAV particle. In some embodiments, the detection reagent binds a conformational epitope on the AAV capsid.

In some embodiments, the detection reagent comprises a detectable moiety, e.g., one capable of being measured by spectrophotometry (color change), light scattering, optical absorbance, fluorescence, chemiluminescence, electrochemiluminescence, bioluminescence, phosphorescence, radioactivity, magnetic field, or combination thereof. In some embodiments, the detection reagent comprises a binding partner of a detectable moiety. In some embodiments, the detection reagent and detectable moiety each comprises a binding partner in a binding pair such as, e.g., a receptor-ligand pair, complementary oligonucleotides, or cross-reactive moieties such as, e.g., thiol and maleimide or iodoacetamide; aldehyde and hydrazide; or azide and alkyne or cycloalkyne. In some embodiments, the detection reagent comprises biotin, and the detectable moiety comprises avidin or streptavidin. In some embodiments, the detectable moiety comprises horseradish peroxidase (HRP), alkaline phosphatase (AP), glucose oxidase (GO), or beta galactosidase (BGAL or β-gal). In some embodiments, the detection reagent comprises biotin, and the detectable moiety comprises HRP conjugated to avidin or streptavidin.

In some embodiments, the capture reagent is lyophilized. In some embodiments, the capture reagent is provided in solution. In some embodiments, the detection reagent is lyophilized. In some embodiments, the detection reagent is provided in solution. In embodiments where the capture and/or detection reagent is lyophilized, the kit further suitably comprises a buffer for reconstituting or resuspending the lyophilized reagent. In some embodiments, the capture and/or detection reagents are provided separately from other components of the kit, e.g., according to their optimal shipping and/or storage temperatures.

In some embodiments, the kit further comprises one or more of a buffer, an assay stop solution, a calibration reagent, a detectable moiety capable of binding to the detection reagent, and a detectable substrate.

In some embodiments, the kit comprises one or more of an assay buffer, blocking buffer, coating buffer, wash buffer, reconstitution or resuspending buffer, and storage buffer. In some embodiments, the assay buffer comprises phosphate buffered saline, sodium carbonate, sodium bicarbonate, Tris, NaCl, TWEEN, or a combination thereof. In some embodiments, the kit comprises an assay stop solution. In some embodiments, the assay stop solution comprises sulfuric acid.

In some embodiments, the kit comprises a calibration reagent. In some embodiments, the calibration reagent comprises a known quantity of the AAV (e.g., Anc80). In some embodiments, the kit comprises multiple calibration reagents comprising a range of concentrations of the AAV (e.g., Anc80). In some embodiments, the multiple calibration reagents comprise concentrations of the AAV (e.g., Anc80) near the upper and lower limits of quantitation for a method performed using the kit. In some embodiments, the multiple calibration concentrations of the calibration reagents span the entire dynamic range of the method. In some embodiments, the multiple calibration reagents comprise multiple AAV serotypes, e.g., to calibrate the method for detecting different AAV serotypes. In some embodiments, the calibration reagent is a positive control reagent. In some embodiments, the calibration reagent is a negative control reagent. In some embodiments, the calibration reagent is lyophilized. In some embodiments, the calibration reagent is provided in solution.

In some embodiments, the kit comprises a detectable moiety capable of binding to the detection reagent. Detectable moieties are further described herein. In some embodiments, the detectable moiety comprises horseradish peroxidase (HRP), alkaline phosphatase (AP), glucose oxidase (GO), or beta galactosidase (BGAL or β-gal). In some embodiments, the kit further comprises a substrate for the detectable moiety. Exemplary substrates for the detectable moieties are provided herein. In some embodiments, the substrate is 3,3',5,5'-tetramethylbenzidine (TMB), 3,3'-diaminobenzidine (DAB), 2,2'-azino-bis(3-ethylbenzothiazoline-6-sulfonic acid) (ABTS), o-phenylenediamine dihydrochloride (OPD), AMPLEX^{®} Red; 3-amino-9-ethylcarbazole (AEC), homovanillic acid, luminol, and the SUPERSIGNAL^{™} ELISA, QUANTABLU^{™}, and QUANTARED^{™} substrates from ThermoFisher. In some embodiments, the substrate is p-nitrophenyl phosphate (PNPP) and the CDP-STAR^{™} and DYNALIGHT^{™} substrates from ThermoFisher. In some embodiments, the substrate is glucose. In some embodiments, the substrate is o-nitrophenyl-β-D-galactopyranoside (ONPG).

In some embodiments, the kit further comprises one or more of an assay consumable, e.g., assay modules, vials, tubes, liquid handling and transfer devices such as pipette tips, covers and seals, racks, and labels. In some embodiments, the kit further comprises instructions for performing an immunoassay to detect and/or titrate an AAV (e.g., Anc80). In some embodiments, the immunoassay comprises a method described herein.

### EXAMPLES

### Example 1. HPLC Assay (comparative)

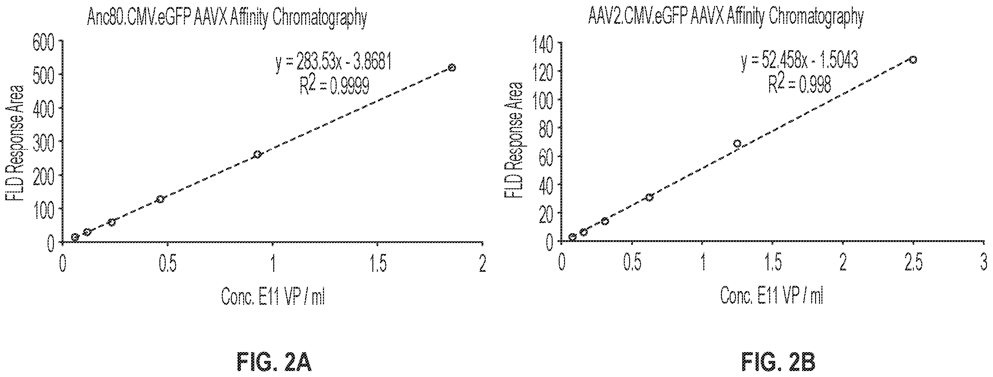

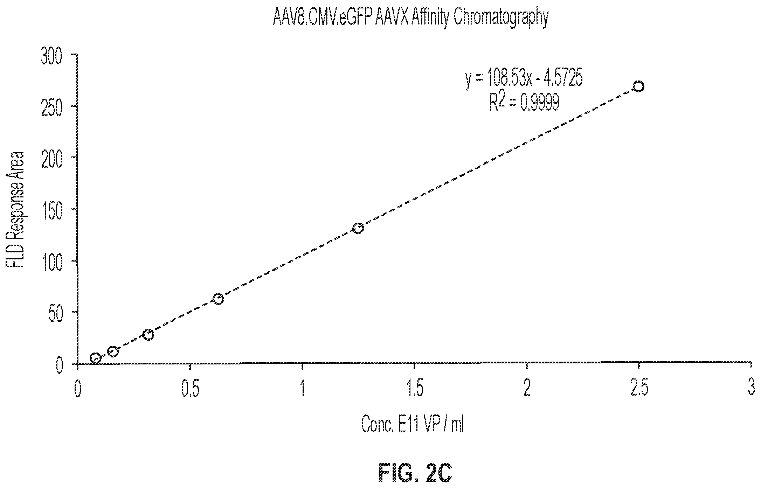

A broad-spectrum AAV (AAVX) affinity column was prepared by packing POROS^{™} CAPTURESELECT^{™} AAVX Affinity Resin (ThermoFisher) into a 50 × 4.6 mm HPLC column by Princeton Chromatography, Inc. An Agilent 1260 Infinity II Bio-Inert LC System with Fluorescence Detector was used to evaluate the AAVX affinity column with various AAV serotype samples, including Anc80.CMV.eGFP, AAV2.CMV.eGFP, and AAV8.CMV.eGFP. As shown in FIGS. 2A-2C, which depict standard curves generated respectively for Anc80.CMV.eGFP, AAV2.CMV.eGFP, and AAV8.CMV.eGFP, response linearity R² > 0.99 was observed for all three AAV serotypes, with a lower limit of detection of approximately 6 × 10⁸ total viral particles.

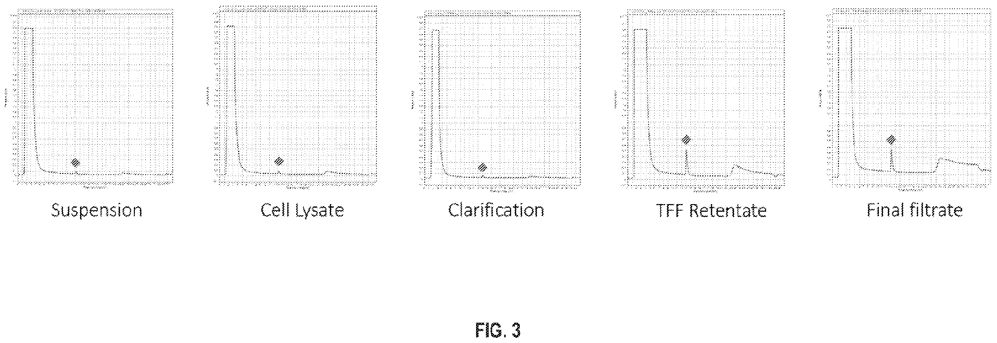

The HPLC method was further tested with in-process samples of Anc80. A 50 L bioreactor producing Anc80.CMV.eGFP was tested using the HPLC method during various steps of the purification process. As shown in FIG. 3, the Anc80.CMV.eGFP capsid peak was successfully detected in all tested steps: cell suspension, cell lysate, after lysate clarification, after tangential flow filtration (TFF), and after the final filtration step.

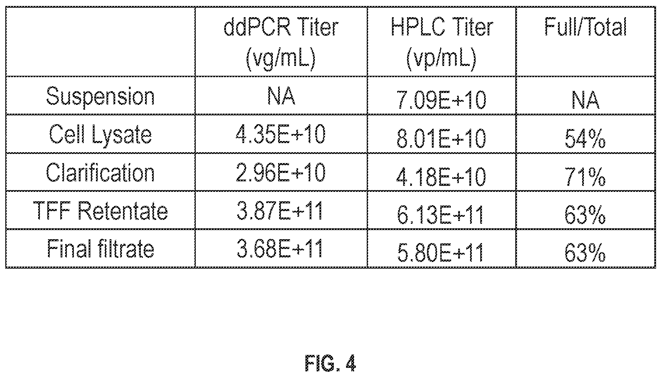

Titers of the in-process Anc80 samples were determined based on linear regression analysis. The titers were also determined using ddPCR, and the full/empty capsid ratios of the in-process samples were calculated as shown in FIG. 4.

### Example 2. Immunoassay

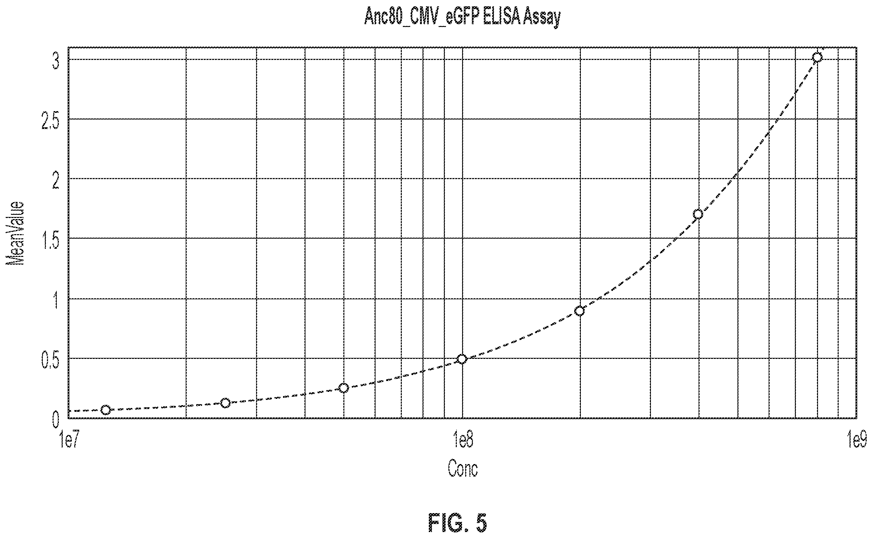

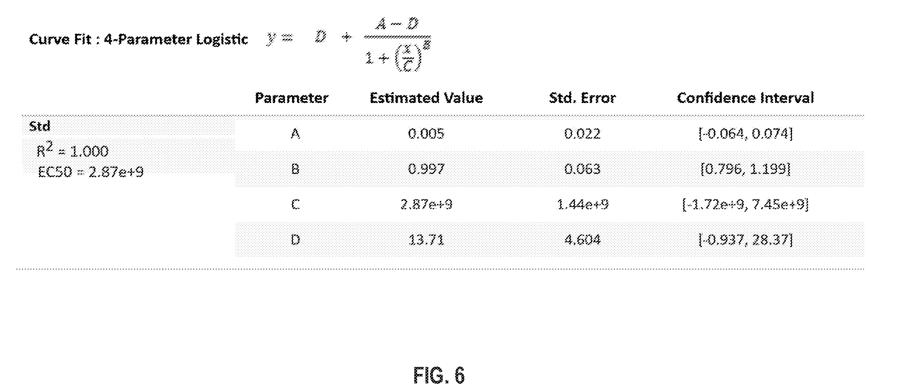

An ELISA assay for Anc80 was developed using an anti-AAV8 capture antibody and the CAPTURESELECT^{™} Biotin Anti-AAVX Conjugate (ThermoFisher) as detection antibody. A standard curve for Anc80.CMV.eGFP was generated using a 4-parameter logistic regression with R² > 0.99 (see FIG. 5, standard curve, and FIG. 6, regression calculation).

The ELISA assay was also applied to in-process samples from a 50 L bioreactor producing Anc80.CMV.eGFP. The titers of in-process samples are measured, and the results are comparable with titers measured by other methods.

## Claims

1. A method for detecting adeno-associated virus serotype Anc80 in a sample, comprising:
(a) contacting the sample with: (i) a capture reagent that specifically binds an adeno-associated virus serotype 8 (AAV8); and (ii) a detection reagent that binds a broad array of adeno-associated virus serotypes (AAVx), wherein each of the capture reagent and the detection reagent is capable of binding the Anc80 and wherein the capture and detection reagent are antibodies or antigen-binding fragments thereof;
(b) forming a binding complex comprising the capture reagent, the Anc80, and the detection reagent; and
(c) detecting the binding complex, thereby detecting the Anc80 in the sample, wherein the capture reagent is immobilized on a surface.

2. The method of claim 1, wherein the capture reagent comprises biotin, and the surface comprises avidin or streptavidin.

3. The method of claim 1 or 2, wherein the surface is a multi-well plate, and the capture reagent is immobilized in a well of the multi-well plate.

4. The method of any one of claims 1 to 3, wherein the capture reagent and/or the detection reagent bind(s) to a capsid protein of the Anc80.

5. The method of claim 4, wherein the capture reagent binds to VP3 of Anc80, preferably wherein the capture reagent binds to an epitope within residues 580 to 600 of the Anc80 VP3, more preferably wherein the capture reagent binds to LQSANT (SEQ ID NO:1).

6. The method of any one of claims 1 to 5, wherein the detection reagent comprises a binding partner of a detectable moiety, optionally wherein the detection reagent comprises biotin, and the detectable moiety comprises horseradish peroxidase conjugated to avidin or streptavidin.

7. A kit for detecting adeno-associated virus serotype Anc80 in a sample, comprising:
(a) a capture reagent that specifically binds an adeno-associated virus serotype 8 (AAV8);
(b) a detection reagent that binds a broad array of adeno-associated virus serotypes (AAVx), and
(c) a capture surface, wherein the capture reagent is immobilized on the capture surface, or wherein the capture reagent is configured to be immobilized to the capture surface,
wherein each of the capture reagent and the detection reagent is capable of binding the Anc80, and wherein the capture and detection reagent are antibodies or antigen-binding fragments thereof.

8. The kit of claim 7, wherein the capture reagent, the detection reagent, or both, are (i) lyophilized or (ii) provided in solution.

9. The kit of claim 7 or 8, wherein the capture reagent comprises biotin, and the capture surface comprises avidin or streptavidin attached thereto.

10. The kit of any one of claims 7 to 9, wherein the capture surface is a multi-well plate, and the capture reagent is immobilized on a well of the multi-well plate.

11. The kit of any one of claims 7 to 10, wherein the capture reagent and/or the detection reagent bind(s) to a capsid protein of the Anc80.

12. The kit of claim 11, wherein the capture reagent binds to VP3 of Anc80, preferably wherein the capture reagent binds to an epitope within residues 580 to 600 of the Anc80 VP3, more preferably wherein the capture reagent binds to LQSANT (SEQ **ID** NO:1).

13. The kit of any one of claims 7 to 12, further comprising a detectable moiety, and the detection reagent comprises a binding partner of the detectable moiety, optionally wherein the detection reagent comprises biotin, and the detectable moiety comprises horseradish peroxidase conjugated to avidin or streptavidin.

14. The kit of any one of claims 7 to 13, further comprising one or more of a buffer, an assay stop solution, a calibration reagent, a detectable moiety capable of binding to the detection reagent, and a detectable substrate.

## Patentansprüche

1. Verfahren zum Nachweisen von Adeno-assoziiertem Virus des Serotyps Anc80 in einer Probe, umfassend:
(a) Inkontaktbringen der Probe mit: (i) einem Fängerreagenz, das spezifisch an ein Adeno-assoziiertes Virus des Serotyps 8 (AAV8) bindet; und (ii) einem Nachweisreagenz, das an ein breites Spektrum von Adeno-assoziierten Virusserotypen (AAVx) bindet, wobei jedes von dem Fängerreagenz und dem Nachweisreagenz in der Lage ist, das Anc80 zu binden, und wobei das Fänger- und Nachweisreagenz Antikörper oder antigenbindende Fragmente davon sind;
(b) Bilden eines Bindungskomplexes, der das Fängerreagenz, das Anc80 und das Nachweisreagenz umfasst; und
(c) Nachweisen des Bindungskomplexes, wodurch das Anc80 in der Probe nachgewiesen wird,
wobei das Fängerreagenz auf einer Oberfläche immobilisiert ist.

2. Verfahren nach Anspruch 1, wobei das Fängerreagenz Biotin umfasst und die Oberfläche Avidin oder Streptavidin umfasst.

3. Verfahren nach Anspruch 1 oder 2, wobei die Oberfläche eine Multiwellplatte ist und das Fängerreagenz in einer Vertiefung der Multiwellplatte immobilisiert ist.

4. Verfahren nach einem der Ansprüche 1 bis 3, wobei das Fängerreagenz und/oder das Nachweisreagenz an ein Kapsidprotein des Anc80 bindet/binden.

5. Verfahren nach Anspruch 4, wobei das Fängerreagenz an VP3 des Anc80 bindet, wobei das Fängerreagenz vorzugsweise an ein Epitop innerhalb der Reste 580 bis 600 des Anc80-VP3 bindet, wobei das Fängerreagenz noch bevorzugter an LQSANT (SEQ ID NO:1) bindet.

6. Verfahren nach einem der Ansprüche 1 bis 5, wobei das Nachweisreagenz einen Bindungspartner einer nachweisbaren Einheit umfasst, wobei das Nachweisreagenz optional Biotin umfasst und die nachweisbare Einheit an Avidin oder Streptavidin konjugierte Meerrettichperoxidase umfasst.

7. Kit zum Nachweisen des Adeno-assoziierten Virusserotyps Anc80 in einer Probe, umfassend:
(a) ein Fängerreagenz, das spezifisch an ein Adeno-assoziiertes Virus des Serotyps 8 (AAV8) bindet;
(b) ein Nachweisreagenz, das ein breites Spektrum von Adeno-assoziierten Virusserotypen (AAVx) bindet, und
(c) eine Fängeroberfläche, wobei das Fängerreagenz auf der Fängeroberfläche immobilisiert ist oder wobei das Fängerreagenz konfiguriert ist, um an der Fängeroberfläche immobilisiert zu werden,
wobei dss Fängerreagenz und dss Nachweisreagenz jeweils in der Lage sind, das Anc80 zu binden, und wobei das Fänger- und Nachweisreagenz Antikörper oder antigenbindende Fragmente davon sind.

8. Kit nach Anspruch 7, wobei das Fängerreagenz, das Nachweisreagenz oder beide (i) lyophilisiert oder (ii) in Lösung bereitgestellt werden.

9. Kit nach Anspruch 7 oder 8, wobei das Fängerreagenz Biotin umfasst und die Fängeroberfläche daran angebrachtes Avidin oder Streptavidin umfasst.

10. Kit nach einem der Ansprüche 7 bis 9, wobei die Fängeroberfläche eine Multiwellplatte ist und das Fängerreagenz an einer Vertiefung der Multiwellplatte immobilisiert ist.

11. Kit nach einem der Ansprüche 7 bis 10, wobei das Fängerreagenz und/oder das Nachweisreagenz an ein Anc80 Kapsidprotein binden/bindet.

12. Kit nach Anspruch 11, wobei das Fängerreagenz an Anc80 VP3 bindet, wobei das Fängerreagenz vorzugsweise an ein Epitop innerhalb der Reste 580 bis 600 des Anc80-VP3 bindet, wobei das Fängerreagenz noch bevorzugter an LQSANT (SEQ ID NO:1) bindet.

13. Kit nach einem der Ansprüche 7 bis 12, weiter umfassend eine nachweisbare Einheit, und wobei das Nachweisreagenz einen Bindungspartner der nachweisbaren Einheit umfasst, wobei das Nachweisreagenz optional Biotin umfasst und die nachweisbare Einheit an Avidin oder Streptavidin konjugierte Meerrettichperoxidase umfasst.

14. Kit nach einem der Ansprüche 7 bis 13, weiter umfassend eins oder mehrere von einem Puffer, einer Assay-Stopplösung, einem Kalibrierreagenz, einer nachweisbaren Einheit, die in der Lage ist, an das Nachweisreagenz zu binden, und einem nachweisbaren Substrat.

## Revendications

1. Procédé de détection de sérotype Anc80 de virus adéno-associé dans un échantillon, comprenant :
(a) la mise en contact de l'échantillon avec : (i) un réactif de capture qui se lie spécifiquement au sérotype 8 de virus adéno-associé (AAV8) ; et (ii) un réactif de détection qui se lie à un large éventail de sérotypes de virus adéno-associé (AAVx), dans lequel chacun du réactif de capture et du réactif de détection est capable de se lier à l'Anc80 et dans lequel les réactifs de capture et de détection sont des anticorps ou des fragments de liaison à l'antigène de ceux-ci ;
(b) la formation d'un complexe de liaison comprenant le réactif de capture, l'Anc80 et le réactif de détection ; et
(c) la détection du complexe de liaison, détectant ainsi l'Anc80 dans l'échantillon,
dans lequel le réactif de capture est immobilisé sur une surface.

2. Procédé selon la revendication 1, dans lequel le réactif de capture comprend de la biotine et la surface comprend de l'avidine ou de la streptavidine.

3. Procédé selon la revendication 1 ou 2, dans lequel la surface est une plaque multipuits, et le réactif de capture est immobilisé dans un puits de la plaque multipuits.

4. Procédé selon l'une quelconque des revendications 1 à 3, dans lequel le réactif de capture et/ou le réactif de détection se lie(nt) à une protéine de capside de l'Anc80.

5. Procédé selon la revendication 4, dans lequel le réactif de capture se lie au VP3 de l'Anc80, de préférence dans lequel le réactif de capture se lie à un épitope dans les résidus 580 à 600 du VP3 de l'Anc80, plus préférablement dans lequel le réactif de capture se lie à LQSANT (SEQ ID NO:1).

6. Procédé selon l'une quelconque des revendications 1 à 5, dans lequel le réactif de détection comprend un partenaire de liaison d'une fraction détectable, éventuellement dans lequel le réactif de détection comprend de la biotine, et la fraction détectable comprend de la peroxydase de raifort conjuguée à l'avidine ou à la streptavidine.

7. Kit de détection de sérotype Anc80 de virus adéno-associé dans un échantillon, comprenant :
(a) un réactif de capture qui se lie spécifiquement à un sérotype 8 de virus adéno-associé (AAV8) ;
(b) un réactif de détection qui se lie à un large éventail de sérotypes de virus adéno-associé (AAVx), et
(c) une surface de capture, dans lequel le réactif de capture est immobilisé sur la surface de capture, ou dans lequel le réactif de capture est configuré pour être immobilisé sur la surface de capture,
dans lequel chacun du réactif de capture et du réactif de détection est capable de se lier à l'Anc80, et dans lequel les réactifs de capture et de détection sont des anticorps ou des fragments de liaison à l'antigène de ceux-ci.

8. Kit selon la revendication 7, dans lequel le réactif de capture, le réactif de détection, ou les deux, sont (i) lyophilisés ou (ii) fournis en solution.

9. Kit selon la revendication 7 ou 8, dans lequel le réactif de capture comprend de la biotine, et la surface de capture comprend de l'avidine ou de la streptavidine qui y est attachée.

10. Kit selon l'une quelconque des revendications 7 à 9, dans lequel la surface de capture est une plaque multipuits, et le réactif de capture est immobilisé sur un puits de la plaque multipuits.

11. Kit selon l'une quelconque des revendications 7 à 10, dans lequel le réactif de capture et/ou le réactif de détection se lie(nt) à une protéine de capside de l'Anc80.

12. Kit selon la revendication 11, dans lequel le réactif de capture se lie au VP3 de l'Anc80, de préférence dans lequel le réactif de capture se lie à un épitope dans les résidus 580 à 600 du VP3 de l'Anc80, plus préférablement dans lequel le réactif de capture se lie à LQSANT (SEQ ID NO:1).

13. Kit selon l'une quelconque des revendications 7 à 12, comprenant en outre une fraction détectable, et le réactif de détection comprend un partenaire de liaison de la fraction détectable, éventuellement dans lequel le réactif de détection comprend de la biotine, et la fraction détectable comprend de la peroxydase de raifort conjuguée à l'avidine ou à la streptavidine.

14. Kit selon l'une quelconque des revendications 7 à 13, comprenant en outre un ou plusieurs éléments parmi un tampon, une solution d'arrêt d'analyse, un réactif d'étalonnage, une fraction détectable capable de se lier au réactif de détection et un substrat détectable.
